(19) Europäisches Patentamt
European Patent Office
Office européen des brevets

(11) Publication number: **0 535 528 A1**

(12) **EUROPEAN PATENT APPLICATION**

(21) Application number: **92116333.3**

(51) Int. Cl.$^5$: **C07D 233/94**, A61K 31/415

(22) Date of filing: **24.09.92**

(30) Priority: **02.10.91 IT MI912612**
**17.09.92 IT MI922141**

(43) Date of publication of application:
**07.04.93 Bulletin 93/14**

(84) Designated Contracting States:
**AT BE CH DE DK ES FR GB GR IE IT LI LU MC NL PT SE**

(71) Applicant: **EURORESEARCH S.r.L.**
**Via Mascheroni 23**
**I-20145 Milano(IT)**

(72) Inventor: **Furlan, Diego**
**Strada 8, no. 37**
**I-20090 S. Felice-Segrate (Milan)(IT)**

(74) Representative: **Gervasi, Gemma, Dr. et al**
**NOTARBARTOLO & GERVASI Srl 33, Viale Bianca Maria**
**I-20122 Milano (IT)**

(54) **Antibacterial 5-Nitro-1-methyl-imidazolyl-3-terbutyl-2-hydroxy-aryl-carbinols.**

(57) Disclosed are 5-nitro-1-methyl-imidazolyl-3-terbutyl-2-hydroxy-aryl-carbinols of general formula (I)

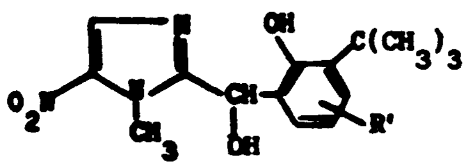

(I)

in which R' is either a linear or branched $C_1$-$C_{10}$ alkyl radical, or - OR'', in which R'' is a linear or branched $C_1$-$C_{10}$ alkyl radical, characterized by having a remarkable antibacterial activity against Helicobapter pylori, a process for their preparation, and relative pharmaceutical compositions as adjuvants in the treatment of gastric ulcer.

EP 0 535 528 A1

## FIELD OF THE INVENTION

The present invention refers to 5-nitro-1-methyl-imidazolyl-3-terbutyl-2-hydroxy-aryl-carbinols, showing a remarkable antibacterial activity against the Helicobapter pylori, as well as a process for their preparation and relative therapeutical compositions containing at least one of these compounds as the active principle, to be used as adjuvants in the treatment of ulcer.

## PRIOR ART

The class of the derivatives of 1-methyl-5-nitro-imidazoline, characterized by the general formula (A):

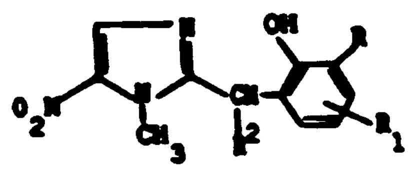

(A)

wherein R is a terbutyl group, $R_1$ is -H and $R_2$ is -OH or

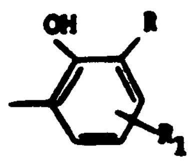

shows a remarkable antibacterial activity against the bacteria Trichomonas vaginalis.

## DISCLOSURE OF THE INVENTION

The Applicant has unexpectedly found out a class of 1-methyl-5-nitro-imidazoline derivatives and precisely the 5-nitro-1-methyl-imidazolyl-3-terbutyl-2-hydroxy-aryl-carbinols, characterized by the following formula (I):

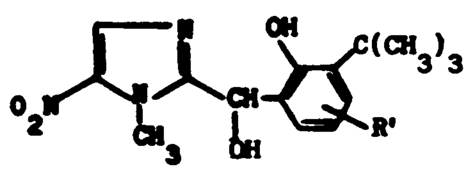

in which R' is either a linear or branched $C_1$-$C_{10}$ alkyl radical, or - OR'', in which R'' is a linear or branched $C_1$-$C_{10}$ alkyl radical, showing a remarkable antibacterial activity against Helicobapter pylori, which is always present in the ulcer pathology.

In particular the Applicant has found out that the compounds having formula (I) exhibit a considerable activity against such bacteria even at a low concentration, whereas other antibacterial agents already known, like for instance metronidazole, proved to be poorly effective.

Because of the peculiar and remarkable antibacterial activity hereinabove, as well as of the low toxicity , the compounds according to the present invention are particularly effective in the human therapy in order to help the treatment of gastric ulcer with conventional drugs.

The present invention further concerns therapeutical compositions containing, as the active principle, therapeutically effective amounts of the compounds having formula (I), in combination with suitable excipients and/or diluents. The therapeutical compositions according to the invention are therefore particularly suitable to be used as adjuvants in the treatment of gastric ulcer.

The present invention further relates to a process for preparing the compound of formula (I), comprising the following steps:

a) reacting the arylhydroxy derivative of formula (II):

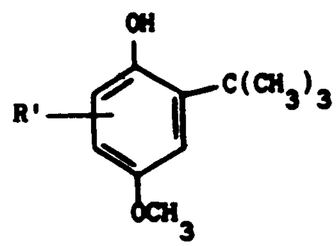

(II)

wherein R' has the above mentioned meanings;
with an alkyl magnesium halide, having formula RMgX, wherein R is alkyl, X is halogen, in a polar anhydrous solvent, at a temperature of from 0° to 15°C, at the reaction triggering, and subsequently at room temperature, in order to obtain the corresponding Grignard reactant having formula (III):

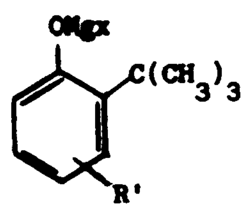

(III)

b) reacting the compound of formula (III) with 5-nitro-1-methyl-2-formyl-imidazole of formula (IV):

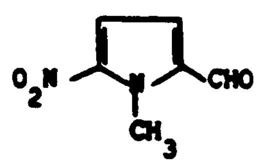

(IV)

in an apolar anhydrous solvent, preferably benzene, at a temperature of from 0° to 15°C at the reaction triggering, and subsequently at room temperature, thereby obtaining the intermediate of formula (V):

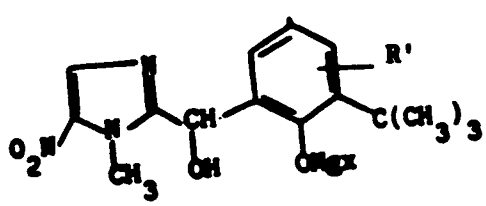

(V)

c) acidifying the reaction solution coming from step (b) up to pH 3.5-4 by means of an aqueous solution of a mineral acid, for hydrolizing the -OMgX group of the intermediate (V).

## DETAILED DESCRIPTION OF THE INVENTION

In the compounds of formula (I) according to the present invention, R' is preferably methyl or terbutyl, while R" is preferably methyl. Particularly preferred compounds according to the present invention are those of formula (I) having

i) R' = $-5-OCH_3$ (EU11100);

ii) R' = $-5-C(CH_3)_3$ (EU11102);

iii) R' = $-5-CH_3$ (EU11103);

iv) R' = $-2-CH_3$ (EU11104);

The Applicant has also unexpectedly found that the compounds of general formula (I) and in particular the above mentioned compound EU11100 has practically no mutagenic activity.

This property together with the above mentioned low toxicity, renders these compounds particularly suitable for long-lasting treatments. In the process according to the present invention step (a) is preferably carried out in ethers such as diethylether, tetrahydrofurane, preferably utilizing as the Grignard reactant the ethyl-magnesium bromide or chloride and step (b) is preferably carried out in benzene and the reaction triggering temperature of this step is preferably comprised between 8 and 12 °C, and step (c) of the process according to the present invention is conducted using as the mineral acid diluted hydrochloric acid.

The therapeutical compositions according to the present invention are suitable to be orally or parenterally administered.

The following examples are hereinbelow provided for illustrative purposes but do not limit in any way the scope of the present invention.

**EXAMPLE 1**

Synthesis of 5-nitro-1-methyl-imidazolyl-3-terbutyl-2-hydroxy-5-methoxy-phenyl-carbinol (EU11100).

In a well dry 2-litre 4 necks flask, provided with a mechanical stirrer, a thermometer, drop funnel and a bubble reflux condenser with a $CaCl_2$ trap, 34.2 g (0.4 mol) of $C_2H_5MgCl$ dissolved in 120 ml of anhydrous THF are poured and a solution consisting of 72 g (0.4 mol) of terbutyl-hydroxyanisole dissolved in 400 ml of anhydrous THF is dripped in 30', in order to avoid that the reaction temperature overcomes 10 °C, while using an effective stirring.

As the dripping is over, the stirring is continued for further 60', by letting the reaction temperature rise up to 20-25 °C.

The thus obtained dark solution is evaporated under vacuum, by keeping the bath temperature ≦ 40 °C. The thus obtained oily residue is then treated with 500 ml of benzene and brought to dryness under vacuum, avoiding that the temperature overcomes 40 °C.

The operation is repeated once again, thus obtaining a pitchy residue which is treated with 500 ml of benzene and stirred at room temperature until obtaining a complete dissolution. At this point, the solution is cooled to 10 °C (inner temperature) and a solution containing 62 g (0.4 mol) of 5-nitro-1-methyl-2-formyl-imidazole dissolved in 400 ml of benzene is added under vigorous stirring, while keeping the inner temperature at 8-12 °C. The dripping does occur in a time of about 30'.

As the addition is over, the reaction mixture is left under stirring for 12 h at room temperature, then a solution of diluted hydrogen chloride is added up to pH 3.5. The thus formed precipitate is filtered and the phases are separated. The organic phase is washed with water, separated, dried on anhydrous $Na_2SO_4$, filtered and evaporated under vacuum until reaching a small volume. The precipitation of a yellowish solid is observed which is filtered and washed with hexane.

The obtained residue is mixed with the first filtered product and a crystallization is carried out from a 60% aqueous solution of ethanol after hot filtering on charcoal. The obtained residue is filtered, washed with cool 60% ethanol and dried under vacuum at a temperature ≦ 40 °C. 50 g of 5-nitro-1-methyl-imidazolyl-3-butyl-2-hydroxy-5-methoxy-phenyl-carbinol are obtained showing the following features:

| | |
|---|---|
| - aspect | Yellow microcrystalline powder |
| - solubility | Soluble in $CH_3OH$, benzene, acetone, ethyl ether; insoluble in water and ligroin. |
| - melting point | 129-131 ° C n.c. |
| - TLC | single spot |
| - IR | consistent |
| - Titer | ≧ 98% (on the dry product) |
| - Drying loss | ≦ 1% |
| - K.F. | ≦ 0.5% |
| - Ash | ≦ 0.2% |
| - Heavy metals | ≦ 20ppm |

The NMR analysis confirm the assigned structure.

**EXAMPLE 2**

Synthesis of 5-nitro-1-methyl-imidazolyl-3,5-diterbutyl-2-hydroxy-phenyl-carbinol (EU11102).

8.88 ml of a 25% solution of ethylmagnesium chloride (25 mmol) in anhydrous THF are introduced under nitrogen atmosphere into a 4 necks flask provided with a mechanical stirrer, a thermometer and a rubber valve for the automatic charge.

30 ml of a solution of the same solvent, containing 5.194 g (25 mmol) of 2.4 diterbutylphenol are added to the previously mentioned solution, by using the above mentioned automatic charge device.

The mixture obtained is then left under stirring for 1 hour at room temperature and the solvent is removed under vacuum.

The residue thus obtained is dissolved in 30 ml benzene, and this solvent is evaporated. This operation is repeated twice, and the solid product thus obtained is treated with 25 ml of benzene and further 25 ml of benzene containing 3.88 g (25 mmol) of 5-nitro-1-methyl-2-formyl-imidazole are added to the mixture obtained in 30 minutes at a temperature comprised between 8 and 12 ° C, by using the above mentioned automatic charge device. This operation is accomplished while maintaining the reaction mixture under vigorous stirring.

When said addition is completed, the mixture is left under stirring at room temperaure for 36 hours.

20 ml of water are added to the brown suspension, 10 % HCl is slowly added up to pH 4, then ethylacetate is added until reaching complete dissolution of the yellow solid obtained.

The organic phase is separated and washed with 10 ml of a saturated solution of sodium carbonate, and finally with 10 ml of water. The organic phase is extracted and dried on anhydrous sodium sulfate.

After filtering hydrated sodium sulfate, the solvent is evaporated, thereby obtaining 8.75 g of a yellow solid consisting of a raw reaction product, corresponding to a material balance of 97% if calculated on the starting phenol. The mixture is then analyzed by HPLC. The analytical yield in the final product results in 70%, with a conversion in imidazole of 95%.

The reaction product is recovered by crystallization, which is carried out according to the following method: 5 g of the raw product are suspended in 60 ml of 60 % ethanol. 15 ml of 98% ethanol are then added to the suspension, previously heated under reflux, the mixture obtained is then left under reflux until the complete dissolution of the solid, then 15 ml of water are added.

2.4 g of the product are precipitated from said solution with a HPLC purity degree >98% and with a yield in isolated product of 48 %.

White crystalline powder m.p. 171 ° C.

The elemental and spectral analyses confirm the assigned structure.

**EXAMPLE 3**

Synthesis of 5-nitro-1-methyl-imidazolyl-3 -terbutyl-2-hydroxy-5 methyl-phenyl-carbinol (EU11103).

8.88 ml of a 25% solution of ethylmagnesium chloride (25 mmol) in anhydrous THF are introduced under nitrogen atmosphere into a 4 necks flask provided with a mechanical stirrer, a thermometer and a rubber valve for the automatic charge.

30 ml of a solution of the same solvent, containing 4.103 g (25 mmol) of 4-methyl-2-terbutylphenol are added to the previously mentioned solution, by using the above mentioned automatic charge device.

The mixture obtained is then left under stirring for 1 hour at room temperature and the solvent is removed under vacuum.

The residue thus obtained is dissolved in 30 ml benzene, and this solvent is evaporated under vacuum. This operation is repeated twice, and the solid product thus obtained is treated with 25 ml of benzene and 25 ml of benzene containing 3.88 g (25 mmol) of 5-nitro-1-methyl-2-formyl-imidazole are added to the mixture obtained in 30 minutes at a temperature comprised between 8 and 12 °C, by using the above mentioned automatic charge device. This operation is accomplished while maintaining the reaction mixture under vigorous stirring.

When said addition is completed, the mixture is left under stirring at room temperature for 18 hours.

20 ml of water are added to the brown suspension, 10 % HCl is slowly added up to pH 4, then ethylacetate is added until reaching complete dissolution of the yellow solid obtained.

The organic phase is separated and washed with 10 ml of a saturated solution of sodium carbonate, and finally with 10 ml of water. The organic phase is extracted and dried on anhydrous sodium sulfate.

After filtering hydrated sodium sulfate, the solvent is evaporated, thereby obtaining 7.5 g of a yellow solid consisting of a raw reaction product, corresponding to a material balance of 93.5%, if calculated on the starting phenol. The mixture is then analyzed by HPLC. The analytical yield in the final product results of 85%, with a conversion in imidazole of 90%.

The reaction product is recovered by crystallization which is carried out according to the following method: 4.5 g of the raw product are suspended in 70 ml of 60 % ethanol. 15 ml of 98% ethanol are then added to the suspension previously heated under reflux, the mixture obtained is then left under reflux until the complete dissolution of the solid, 3.7 g of a reaction product are precipitated from said solution with a purity degree of 97.6% and with a yield in isolated product of 82 %.

Yellow crystalline powder m.p. 172 °C.

The elemental and spectral analyses confirm the assigned structure.

**EXAMPLE 4**

Synthesis of 5-nitro-1-methyl-imidazolyl-3 -terbutyl-2-hydroxy-6 methyl-phenyl-carbinol (EU11104).

8.88 ml of a 25% solution of ethylmagnesium chloride (25 mmol) in anhydrous THF are introduced under nitrogen atmosphere into a 4 necks flask provided with a mechanical stirrer, a thermometer and a rubber valve for the automatic charge, 30 ml of a solution of the same solvent, containing 4.103 g (25 mmol) of 2-methyl-6-terbutylphenol are added to the previously mentioned solution, by using the above mentioned automatic charge device.

The mixture obtained is then left under stirring for 1 hour at room temperature and the solvent is removed under vacuum.

The residue thus obtained is dissolved in 30 ml benzene, and this solvent is evaporated. This operation is repeated twice, and the solid product thus obtained is treated with 25 ml of benzene and 25 ml of benzene containing 3.88 g (25 mmol) of 5-nitro-1-methyl-2-formyl-imidazole are added to the mixture obtained in 30 minutes at a temperature comprised between 8 and 12 °C, by using the above mentioned automatic charge device. This operation is accomplished while maintaining the reaction mixture under vigorous stirring.

When said addition is completed, the mixture is left under stirring at room temperature for 48 hours.

20 ml of water are added to the brown suspension, 10 % HCl is slowly added up to pH 4, then ethylacetate is added until reaching complete dissolution of the yellow solid obtained.

The organic phase is separated and washed with 10 ml of a saturated solution of sodium carbonate, and finally with 10 ml of water. The organic phase is extracted and dried on anhydrous sodium sulfate.

After filtering hydrated sodium sulfate, the solvent is evaporated, thereby obtaining 7.76 g of a yellow solid consisting of a raw reaction product. The mixture is then analyzed by HPLC. The analytical yield in the final product results in 38.1%, with a conversion in imidazole of 65%.

The reaction product is not recovered by crystallization.

White micro crystalline powder m.p. 173 °C.

The elemental and spectral analyses confirm the assigned structure.

**ORAL AND INTRAPERITONEAL ACUTE TOXICITY OF THE COMPOUNDS EU11100, EU11102, EU11103, EU11104 IN MICE**

20 Swiss female albino mice are used for this test of the average weight of 20 g (±2), coming from Nossan breeding .

Stabling and environmental conditions

Cages: in Makrolon® with saw dust litter,
Number of animals per cage: 5
Acclimation period: 1 week
Temperature: 22° C (± 2° C)
Humidity : 60%,
Diet: pellets for mice produced by Nossan firm,
Water ad libitum
Fast before the treatment: 16 hours

Administration route

Oral by gastric route and intraperitoneal.

Substances

The substances to be studied: EU11100, EU11102, EU11103, EU11104 are treated with 0.5% carboxymethylcellulose in spring water, for the oral administration, whereas the same substances are intraperitoneally administered in the form of a physiological solution .

For each of the above mentioned substances, a mother suspension is prepared having a concentration in the active ingredient of 250 mg/ml for the oral administration, and analogously a mother suspension is prepared for the intraperitoneal administration having a concentration of the desired active principle of 100 mg/ml.

Administered doses

The following doses are used
a) oral route: 5000 mg/kg
b) intraperitoneal route : 2000 mg/kg

Other administered doses

If mortality is observed at the above mentioned doses, other doses are administered in order to obtain a dose-response curve.

Number of animals per dose.

5 animals are used for every utilized concentration.

Period of observation

After the administration of the above substances the animals are kept under observation for 14 days , during which the mortality and the behaviour is observed.

$LD_{50}$ evaluation

$LD_{50}$ is determined on the base of the cumulative mortality observed in the successive 14 days after the administration by using the Probits method.

The results for the 4 compounds EU11100, EU11102, E011103, EU11104 are reported in the respective tables 1,2,3 and 4.

TABLE N. 1

Acute toxicity $LD_{50}$ and observations on the behaviour of Swiss female mice per os and ip treated with EU 11102

| Route | Dose mg/Kg | N. animals | Mortality per day | | | | | | | | | | | | | | Observation on the behaviour |
|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | |
| os | 5000 | 5 | 0 | 0 | 0 | 0 | 0 | 0 | 0 | 0 | 0 | 0 | 0 | 0 | 0 | 0 | No alteration |
| $LD_{50}$ os > 5000 mg/Kg | | | | | | | | | | | | | | | | | |
| ip | 2000 | 5 | 0 | 0 | 0 | 0 | 0 | 0 | 0 | 0 | 0 | 0 | 0 | 0 | 0 | 0 | Abdominal spasms eight increase in motor activity<br>After 2 h. normal behaviour |
| $LD_{50}$ i.p.> 2000 mg/Kg | | | | | | | | | | | | | | | | | |

TABLE N. 2

Acute toxicity $LD_{50}$ and observations on the behaviour of Swiss female mice orally treated with EU 11102.

| Route | Dose mg/Kg | N. animals | Mortality per day | | | | | | | | | | | | | | Observation on the behaviour |
|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | |
| os | 5000 | 5 | 0 | 0 | 0 | 0 | 0 | 0 | 0 | 0 | 0 | 0 | 0 | 0 | 0 | 0 | No alteration |
| $LD_{50}$ os > 5000 mg/Kg | | | | | | | | | | | | | | | | | |
| ip | 2000 | 5 | 0 | 0 | 0 | 0 | 0 | 0 | 0 | 0 | 0 | 0 | 0 | 0 | 0 | 0 | Abdominal spasms light increase in motor activity<br>After 2 h. normal behaviour |
| $LD_{50}$ i.p. > 2000 mg/Kg | | | | | | | | | | | | | | | | | |

**TABLE N. 3**

Acute toxicity $LD_{50}$ and observations on the behaviour of Swiss female mice orally treated with EU 11104

| Route | Dose mg/Kg | N. animals | Mortality per day | | | | | | | | | | | | | | Observation on the behaviour |
|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | |
| os | 5000 | 5 | 0 | 0 | 0 | 0 | 0 | 0 | 0 | 0 | 0 | 0 | 0 | 0 | 0 | 0 | No alteration |
| $LD_{50}$ os > 5000 mg/Kg | | | | | | | | | | | | | | | | | |
| ip | 2000 | 5 | 0 | 0 | 0 | 0 | 0 | 0 | 0 | 0 | 0 | 0 | 0 | 0 | 0 | 0 | Abdominal spasms light increase in motor activity<br>After 2 h. normal behaviour |
| $LD_{50}$ i.p. > 2000 mg/Kg | | | | | | | | | | | | | | | | | |

**TABLE N. 4**

Acute toxicity $LD_{50}$ and observations on the behaviour of Swiss female mice per os and ip treated with EU 11100

| Route | Dose mg/Kg | N. animals | Mortality per day | | | | | | | | | | | | | | Observation on the behaviour |
|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | |
| os | 5000 | 5 | 0 | 0 | 0 | 0 | 0 | 0 | 0 | 0 | 0 | 0 | 0 | 0 | 0 | 0 | No alteration |
| $LD_{50}$ os > 5000 mg/Kg | | | | | | | | | | | | | | | | | |
| ip | 2000 | 5 | 5 | | | | | | | | | | | | | | Doses: 2000 - 1500 - 1000 mg/Kg: after 15-30' considerable reduction motor activity and subsequent loss of body righting reflex. Death within 6-24 h. |
| | 1500 | 5 | 5 | | | | | | | | | | | | | | |
| | 1000 | 5 | 3 | | | | | | | | | | | | | | |
| | 750 | 5 | 1 | | | | | | | | | | | | | | 750 mg/Kg: motor activity reduction |
| | 500 | 5 | 0 | | | | | | | | | | | | | | 500 mg/Kg: no alteration |
| $LD_{50}$ i.p.= 890 mg/Kg (confidence limits: 781 - 1014) | | | | | | | | | | | | | | | | | |

As it results by the data reported in the above tables, the compounds EU11102, EU11103, EU11104, when orally administered are not toxic at the dose of 5000 mg/kg and when intraperitoneally administered are not toxic at the dose of 2000 mg/kg.

The compound EU11100 when orally administered is not toxic at the dose of 5000 mg/kg, at the dose of 2000, 1500, and 1000 mg/kg when intraperitoneally administered, results toxic, with a mortality of 100%

at 1500 and at 2000 mg/kg, and with considerable behaviouristic alteration, such as the loss of body-righting reflex at all the three doses above mentioned.

$LD_{50}$ for EU11100 by intraperitoneal route results 890 mg/kg

**ACTIVITY AGAINST HELICOBAPTER PYLORI**

A) INHIBITION HALOS OF THE BACTERIAL GROWTH

METHOD

Substances under examination: EU11100, EU11102, EU11103, EU11104.

Comparison substance: Metronidazole

Solubilization of the substances : All the substances to be studied are dissolved at room temperature in DMSO.

Preparation of the disks

Some commercially available antibiogram disks are so impregnated with the above mentioned compounds that each disk has concentration equal to 10, 1, 0.1, 0.01 µg.

Used bacterial strains:

10 strains of Helicobapter pylori are used of recent clinical isolation.

Medium used for the test:

Columbia Blood Agar Base is used, containing 5% of defibrinated horse blood, 2 % of calf serum, 2 vials of Vitox per liter of medium and Vancomicine (10 µg/ml) Trimetoprim lactate(5 µg/ml) and Polimixin B (2.5 U/ml).

Execution of the text:.

0.1 ml of a suspension of the different used strains are uniformly distributed onto Petri plates, containing 20 ml of Columbia Blood Agar base, obtained by taking a sample, by means of a platinum loop, of different colonies from a 48 hours culture, grown in anaerobiosis jars with "Gas Pack", without a catalyst and diluted into a physiological solution. The obtained suspension shows an optical density corresponding to $10^{10}$ UFC/ml.

4 disks per plate, previously impregnated with the substances under examination, are then placed onto the agar layer of each plate.

After 48 hours of anaerobiosis incubation, as above described, the inhibition halos of the bacterial growth, produced by the different activity of the substances inside the agar, are evaluated. The results are reported on the following Table 5.

TABLE n. 5

Inhibition halos of Helicobacter Pylors, produced by the substances: EU11102 - EU11103 - EU11104 EU11100 in comparison with Metronidazole.

| Strain N. | Inhibition halo (mm) at the concentration (µg/disk) | | | | | | | | | | | | | | | | | | | |
|---|---|---|---|---|---|---|---|---|---|---|---|---|---|---|---|---|---|---|---|---|
| | EU 11102 | | | | EU 11103 | | | | EU 11104 | | | | EU 11100 | | | | Metronidazole | | | |
| | 10 | 1 | 0.1 | 0.01 | 10 | 1 | 0.1 | 0.01 | 10 | 1 | 0.1 | 0.01 | 10 | 1 | 0.1 | 0.01 | 10 | 1 | 0.1 | 0.01 |
| 1 | 23 | 15 | 0 | 0 | 40 | 30 | 18 | 0 | 33 | 27 | 18 | 0 | 50 | 35 | 26 | 0 | 45 | 0 | 0 | 0 |
| 2 | 22 | 16 | 0 | 0 | 38 | 25 | 0 | 0 | 30 | 22 | 0 | 0 | 44 | 30 | 18 | 0 | 33 | 0 | 0 | 0 |
| 3 | 22 | 12 | 0 | 0 | 35 | 24 | 0 | 0 | 28 | 22 | 0 | 0 | 38 | 30 | 14 | 0 | 45 | 0 | 0 | 0 |
| 4 | 24 | 16 | 0 | 0 | 44 | 36 | 20 | 0 | 36 | 30 | 18 | 0 | 50 | 38 | 26 | 0 | 42 | 0 | 0 | 0 |
| 5 | 22 | 16 | 0 | 0 | 22 | 17 | 0 | 0 | 28 | 22 | 0 | 0 | 38 | 22 | 12 | 0 | 42 | 0 | 0 | 0 |
| 6 | 25 | 17 | 0 | 0 | 43 | 32 | 10 | 0 | 35 | 29 | 14 | 0 | 48 | 34 | 22 | 0 | 41 | 0 | 0 | 0 |
| 7 | 21 | 14 | 0 | 0 | 28 | 20 | 0 | 0 | 26 | 22 | 0 | 0 | 35 | 28 | 12 | 0 | 32 | 0 | 0 | 0 |
| 8 | 23 | 15 | 0 | 0 | 45 | 37 | 21 | 0 | 37 | 31 | 17 | 0 | 48 | 38 | 21 | 0 | 40 | 0 | 0 | 0 |
| 9 | 24 | 16 | 0 | 0 | 33 | 22 | 0 | 0 | 30 | 21 | 0 | 0 | 43 | 28 | 10 | 0 | 38 | 0 | 0 | 0 |
| 10 | 23 | 15 | 0 | 0 | 38 | 24 | 0 | 0 | 32 | 23 | 0 | 0 | 43 | 28 | 14 | 0 | 37 | 0 | 0 | 0 |
| Average | 22.9 | 15.1 | | | 36.6 | 26.7 | | | 31.5 | 24.9 | | | 43.7 | 30.6 | | | 39.5 | | | |
| +/-S.E. | 0.37 | 0.43 | | | 2.31 | 2.13 | | | 1.17 | 1.23 | | | 1.60 | 1.33 | | | 1.42 | | | |

As it clearly results from the experimental data above reported, the substances EU11102, EU11103, EU11104 are active against all the Helicobapter pylori strains used.

Actually, the most active compound is EU11103 with an average halo at the concentration of 10 µg/disk equal to 36.6 mm, if compared to the 31.5 mm of EU11104 and to the 22.9 mm of EU11102.

At the concentration of 1 µg/disk all three compounds EU11102, EU11103, EU11104 result active against all the Helicobapter pylori used, and also in this case the highest activity is observed for the compound EU11103.

At the concentration of 0.1 µg the substances EU11103 and EU11104 result active against 4 out of 10 strains.

In any case the inhibition halos produced from the above mentioned compounds result smaller than those caused by the molecule EU11100, which even at the dose of 0.1 µg/disk is still able to produce halos against all the strains used.

On the contrary Metronidazole, although producing at the maximum concentration used (10 µg/disk) inhibition halos similar to those of EU11100 and larger than those of EU11102, EU11103, EU11104, is inactive at the concentrations of 1 and 0.1 µg/disk while at the same concentrations all 4 substances are still active.

B) MINIMAL INHIBITING CONCENTRATION

METHOD

Substances under examination: EU11100, EU11102, EU11103, EU11104.

Comparison substance: Metronidazole

Solubilization of the substances: All the substances to be studied are dissolved at room temperature in DMSO.

Preparation of the disks

Some commercially available antibiogram disks are so impregnated with the above mentioned compounds that each disk has concentration equal to 10, 1, 0.1, 0.01 µg.

Used bacterial strains:

10 strains of Helicobapter pylori are used of recent clinical isolation

Medium used for the test:

Columbia Blood Agar Base is used, containing 5% of defibrinated horse blood, 2 % of calf serum, 2 vials of Vitox per liter of medium and Vancomicine (10 µg/ml) Trimetoprim lactate (5 µg/l) and Polimixin B (2.5 U/ml).

Execution of the text

The compounds to be analyzed and the blank (Metronidazole) are diluted, starting from the initial concentration of 32 µg/ml agar, with an equal volume of a nutritive medium, maintained liquid at 45 °C and then poured, in a volume of 20 ml, into plates having a 20 cm diameter, until concentration of 0.007 µg/ml is reached.

The sowing of the bacteria, prepared as described in the preceding test is carried out by means a "multi-point inoculator".

After 48 hours incubation under the condition described in the preceding test, the plates are examined and the Minimal Inhibiting Concentration is evaluated, namely that dose corresponding to growth absence.

In table 6 reported are the experimental data of said test:

TABLE 6 : Minimal inhibiting activity of the molecule EU11100, EU11102, EU11103, and EU11104 in comparison with Metronidazole (blank)

| Strain No | M.I.C. (µg/ml) | | | | |
|---|---|---|---|---|---|
| | EU11102 | EU11103 | EU11104 | EU11100 | Metronidazole |
| 1 | 0.5 | 0.06 | 0.12 | 0.12 | 0.25 |
| 2 | 0.25 | 0.06 | 0.03 | 0.03 | 0.06 |
| 3 | 0.25 | 0.03 | 0.06 | 0.03 | 0.25 |
| 4 | 0.5 | 0.03 | 0.015 | 0.03 | 0.25 |
| 5 | 0.25 | 0.03 | 0.03 | 0.015 | 0.015 |
| 6 | 0.5 | 0.03 | 0.03 | 0.03 | 0.12 |
| 7 | 0.25 | 0.06 | 0.06 | 0.06 | 0.25 |
| 8 | 0.25 | 0.06 | 0.06 | 0.03 | 0.25 |
| 9 | 0.25 | 0.03 | 0.06 | 0.03 | 0.12 |
| 10 | 0.25 | 0.015 | 0.015 | 0.03 | 0.12 |
| MIC 50% | 0.25 | 0.03 | 0.03 | 0.03 | 0.12 |
| MIC 90% | 0.5 | 0.06 | 0.06 | 0.06 | 0.25 |

| Compound | MIC range (µg/ml) |
|---|---|
| EU11102: | 0.25 - 0.5 |
| EU11103: | 0.015 - 0.016 |
| EU11104 | 0.015 - 0.016 |
| EU11100 | 0.015 - 0.016 |
| Metronidazole: | 0.015 - 0.25 |

As it results from the above data EU11102 exhibits a fairly good inhibiting activity comprised between 0.25 and 0.5.

The other three molecules EU11103, EU11104, EU11100 exhibit very low MIC values, and in any case much lower than those of Metronidazole, and consequently they are much more active than the comparison substance.

**MUTAGENIC ACTIVITY OF COMPOUND EU11100 IN SALMONELLA TYPHIMURIUM.**

**AMES TEST.**

The used concentrations of the compound EU11100 are respectively 250, 500 and 1000 µg/capsule in the test with metabolic activation and 250 and 500 µg/capsule in the test without metabolic activation (consistently with the survival of the Salmonella strains). The used Salmonella strains are as follows: TA-1535, TA-1537, TA-1538, TA-98, TA-100.

The experimental data obtained by carrying out said test are reported in the following table 7:

TABLE 7-AMES TEST

A-WITHOUT DIRECT MUTATION

(µg/capsule)

| Strain | 0.0 | 250 | 500 |
|---|---|---|---|
| TA-1535 | 23 | 25 | 28 |
| TA-1537 | 10 | 12 | 11 |
| TA-1538 | 22 | 18 | 20 |
| TA-98 | 19 | 25 | 32 |
| TA-100 | 145 | 241 | 283 |

B- WITH DIRECT MUTATION

(µg/capsule)

| Strain | 0.0 | 250 | 500 | 1000 |
|---|---|---|---|---|
| TA-1535 | 26 | 32 | 22 | 30 |
| TA-1537 | 15 | 16 | 12 | 12 |
| TA-1538 | 32 | 31 | 25 | 28 |
| TA-98 | 34 | 32 | 27 | 34 |
| TA-100 | 196 | 174 | 202 | 202 |

The data obtained, as reported in the above table, prove that EU11100 does not increase the spontaneous reversion of Salmonella, where also the metabolic activation is used. In the test without metabolic activation a doubling of the base value (1.95 times) is observed for the strain TA-100 at concentrations of 500 µg/capsule, whereas other strains do not give rise to any particular signal. The

concentration of 1000 µg/capsule, in the absence of metabolic activation, is found to be toxic.

**DIRECT MUTATION IN THE POMBE SYNDROME**

The tests are carried out on the compound EU11100 with or without metabolic activity for a treatment period of 16 h in the growth step. The used concentrations are 0.25-0.50 mg/ml without metabolic activation and 0.075-0.150 mg/ml with metabolic activation. The experimental results are reported hereinbelow in the following TABLE 8.

TABLE 8-POMBE SYNDROME

(16 h treatment)

A- WITHOUT DIRECT MUTATION

| | Frequency of: | |
|---|---|---|
| Concentration | Mutation | Survival (%) |
| 0.00 | 2.28 | 100 |
| 0.25 | 1.60 | 71.1 |
| 0.50 | 0.00 | 36.4 |

B- WITH DIRECT MUTATION

| | Frequency of: | |
|---|---|---|
| Concentration | Mutation | Survival (%) |
| 0.00 | 1.60 | 100 |
| 0.075 | 2.11 | 89.9 |
| 0.150 | 3.22 | 29.5 |

The results obtained reported in the above Table 8 demonstrate that the compound of formula (I) does not increase the number of mutants with respect to the blank.

## Claims

1. 5-nitro-1-methyl-imidazolyl-3-terbutyl-2-hydroxy-aryl- carbinols having the following formula (I):

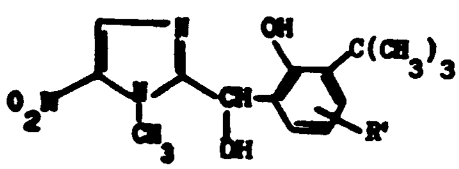

(I)

in which R' is either a linear or branched $C_1$-$C_{10}$ alkyl radical, or - OR", in which R" is a linear or branched $C_1$-$C_{10}$ alkyl radical.

2. The 5-nitro-1-methyl-imidazolyl-3-terbutyl-2-hydroxy-aryl carbinols according to claim 1 wherein wherein R' is selected from methyl or terbutyl, and when R' is OR", R" is methyl.

3. The 5-nitro-1-methyl-imidazolyl-3-terbutyl-2-hydroxy-aryl-carbinol according to claim 2 wherein R' = -5-$OCH_3$.

4. The 5-nitro-1-methyl-imidazolyl-3-terbutyl-2-hydroxy-aryl carbinol according to claim 2 wherein R' = -5-$C(CH_3)_3$.

5. The 5-nitro-1-methyl-imidazolyl-3-terbutyl-2-hydroxy-aryl-carbinol according to claim 2 wherein R' = -5-$CH_3$.

6. The 5-nitro-1-methyl-imidazolyl-3-terbutyl-2-hydroxy-aryl-carbinol according to claim 2 wherein R' = -2-$CH_3$.

7. A process for preparing 5-nitro-1-methyl-imidazolyl-3-terbutyl-2-hydroxy-aryl -carbinols having the following formula (I):

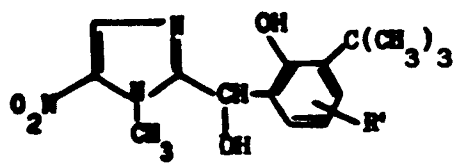

(I)

in which R' is either a linear or branched $C_1$-$C_{10}$ alkyl radical, or - OR", in which R" is a linear or branched $C_1$-$C_{10}$ alkyl radical, comprising the following steps:
a) reacting the arylhydroxy derivative of formula (II):

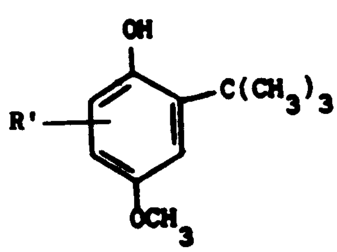

(II)

wherein R' has the above mentioned meanings;
with an alkyl magnesium halide, having formula RMgX wherein R is alkyl, X is halogen, in a polar anhydrous solvent, at a temperature of from 0° to 10°C, at the reaction triggering, and subsequently at room temperature, in order to obtain the corresponding Grignard reactant having formula (III):

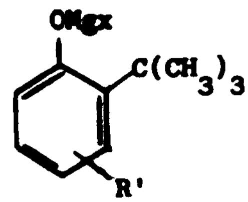

(III)

b) reacting the compound of formula (III) with 5-nitro-1-methyl-2-formyl-imidazole of formula (IV):

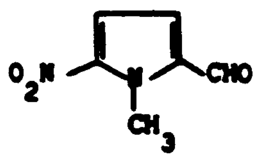

(IV)

in an apolar anhydrous solvent, preferably benzene, at a temperature of from 0° to 15°C at the reaction triggering, and subsequently at room temperature, in order to obtain the intermediate of formula (V):

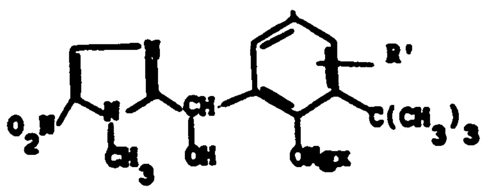

(V)

c) acidifying the reaction solution coming from step (b) up to pH 3.5-4 by means of an aqueous solution of a mineral acid, for hydrolizing the -OMgX group of the intermediate (V).

**8.** The process according to claim 7, wherein step (a) is conducted in ethers selected from diethylether, tetrahydrofurane, utilizing as the Grignard reactant the ethyl-magnesium bromide or chloride and step (b) is carried out in benzene and the reaction triggering temperature of this step is comprised between 8 and 12 °C, and step (c) of the process according to the present invention is conducted, using as the mineral acid, diluted hydrochloric acid.

**9.** Therapeutical compositions containing, as the active principle, therapeutical effective amounts of 5-nitro-1-methyl-imidazolyl-3-terbutyl-2-hydroxy-aryl-carbinols, having the following formula (I):

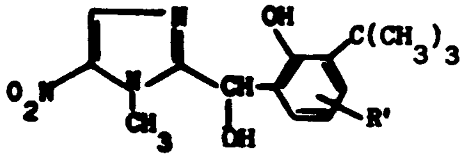

(I)

in which R' is either a linear or branched $C_1$-$C_{10}$ alkyl radical, or - OR", in which R" is a linear or branched $C_1$-$C_{10}$ alkyl radical, in combination with suitable excipients and/or diluents, as adjuvants for the treatment of ulcer pathology.

**10.** The therapeutical compositions according to claim 9 containing the active principle of formula (I) wherein R' = $-5-OCH_3$.

**11.** The therapeutical compositions according to claim 9 containing the active principle of formula (I) wherein R' = $-5-C(CH_3)_3$.

**12.** The therapeutical compositions according to claim 9 containing the active principle of formula (I) wherein R' = $-5-CH_3$.

**13.** The therapeutical compositions according to claim 9 containing the active principle of formula (I) wherein R' = $-2-CH_3$.

**14.** The therapeutical compositions according to claim 9, suitable for the oral or parenteral administration.

European Patent Office

# EUROPEAN SEARCH REPORT

Application Number

EP 92 11 6333

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 063 401 (BONOBI LORIS JACOPO) | | C07D233/94<br>A61K31/415 |
| A | EP-A-0 103 100 (FARMATIS S.R.L.,CORSO EUROPA N.S.) | | |
| A | EP-A-0 111 657 (TESSITORE PIETRO TOMMASO) | | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 22 DECEMBER 1992 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document